# FASCICULE DE BREVET EUROPEEN

(11) **EP 3 317 400 B1**
(45) Date de publication et mention de la délivrance du brevet: **28.10.2020**
(21) Numéro de dépôt: 16742353.2
(22) Date de dépôt: 30.06.2016
(51) Int. Cl.: C12N 5/0783, A01N 1/02

(54) **PROCÉDÉ DE CRYOCONSERVATION DE LYMPHOCYTES INFILTRANT LA TUMEUR**
VERFAHREN ZUR KRYOKONSERVIERUNG VON TUMORINFILTRIERENDEN LYMPHOZYTEN
METHOD FOR THE CRYOPRESERVATION OF TUMOUR-INFILTRATING LYMPHOCYTES

(30) Priorité: 30.06.2015 FR 1556164
(43) Date de publication de la demande: 09.05.2018
(73) Titulaire: Chu Nantes, 44000 Nantes (FR)
(72) Inventeur: DE LARICHAUDY, Joffrey, 92240 Malakoff (FR)
(74) Mandataire: Plasseraud IP
(86) Numéro de dépôt international: PCT/FR2016/051631
(87) Numéro de publication internationale: WO 2017/001784

(56) Documents cités:
- ANTHONY VISIONI ET AL: "Expansion of melanoma-specific T cells from lymph nodes of patients in stage III: Implications for adoptive immunotherapy in treating cancer", SURGERY, vol. 152, no. 4, 1 octobre 2012 (2012-10-01), pages 557-566, XP055132912, ISSN: 0039-6060, DOI: 10.1016/j.surg.2012.07.002
- IKARASHI H ET AL: "Solid-phase anti-CD3 antibody activation and cryopreservation of human tumor-infiltrating lymphocytes derived from epithelial ovarian cancer", JAPANESE JOURNAL OF CANCER RESEARCH, JAPANESE CANCER ASSOCIATION, TOKYO, JP, vol. 83, no. 12, 1 décembre 1992 (1992-12-01), pages 1359-1365, XP009186820, ISSN: 0910-5050
- GOLAB KAROLINA ET AL: "Challenges in cryopreservation of regulatory T cells (Tregs) for clinical therapeutic applications", INTERNATIONAL IMMUNOPHARMACOLOGY, ELSEVIER, AMSTERDAM, NL, vol. 16, no. 3, 18 février 2013 (2013-02-18), pages 371-375, XP028561441, ISSN: 1567-5769, DOI: 10.1016/J.INTIMP.2013.02.001
- THUMANN P ET AL: "Antigen loading of dendritic cells with whole tumor cell preparations", JOURNAL OF IMMUNOLOGICAL METHODS, ELSEVIER SCIENCE PUBLISHERS B.V.,AMSTERDAM, NL, vol. 277, no. 1-2, 1 juin 2003 (2003-06-01), pages 1-16, XP004430542, ISSN: 0022-1759, DOI: 10.1016/S0022-1759(03)00102-9
- A. SMAGUR ET AL: "Comparison of the cryoprotective solutions based on human albumin vs. autologous plasma: its effect on cell recovery, clonogenic potential of peripheral blood hematopoietic progenitor cells and engraftment after autologous transplantation", VOX SANGUINIS., vol. 108, no. 4, 6 mars 2015 (2015-03-06), pages 417-424, XP055223133, CH ISSN: 0042-9007, DOI: 10.1111/vox.12238
- A. SMAGUR ET AL: "Impact of different dimethyl sulphoxide concentrations on cell recovery, viability and clonogenic potential of cryopreserved peripheral blood hematopoietic stem and progenitor cells", VOX SANGUINIS., vol. 104, no. 3, 9 octobre 2012 (2012-10-09), pages 240-247, XP055223173, CH ISSN: 0042-9007, DOI: 10.1111/j.1423-0410.2012.01657.x
- BISSOYI AKALABYA ET AL: "Effects of non-toxic cryoprotective agents on the viability of cord blood derived MNCs", CRYO LETTERS, CRYOLETTERS LLP, UK, vol. 34, no. 5, 1 septembre 2013 (2013-09-01), pages 453-465, XP009186819, ISSN: 0143-2044

## Description

La présente invention est définie par les revendications. Tout objet sortant de la portée des revendications n'est fourni qu'à des fins d'information uniquement. La présente description divulgue un procédé de cryoconservation d'au moins un échantillon de lymphocytes infiltrant la tumeur, comprenant les étapes suivantes :
i) culture *in vitro* de lymphocytes infiltrant la tumeur à partir de prélèvement de nodules cutanés en transit, ganglion ou métastase d'un patient atteint d'un mélanome de stade 3 ou 4, comprenant des étapes spécifiques,
ii) mélange d'au moins un échantillon obtenu à l'étape i) avec une composition comprenant, dans un milieu physiologiquement acceptable :
   a) de la sérum albumine humaine,
   b) au moins un saccharide, et
   c) au moins deux ingrédients choisis parmi le DMSO, la L-cystéine, le coenzyme Q10
   et les alcanediols en C3-C5, puis
iii) congélation du mélange obtenu à l'étape ii).

La cryoconservation est un processus dans lequel des échantillons biologiques sont stockés à très basse température. La cryoconservation de matériel biologique est généralement effectuée par la congélation dudit matériel, dans un support approprié, tel qu'un tube ou une ampoule en verre ou en matière plastique (généralement appelé "paillette" ou tube de congélation dans le domaine de la cryoconservation), ledit support étant adapté pour le stockage à long terme et à basse température.

La cryoconservation pose cependant un certain nombre de problèmes et de contraintes techniques. Notamment, des lésions cellulaires peuvent se produire lors de la décongélation, entraînant l'apoptose ou l'éclatement des cellules. En outre, la survie des cellules cryoconservées peut dépendre des conditions et des techniques employées lors de la congélation. Le contrôle de la vitesse de refroidissement est important : un refroidissement à faible vitesse permet une cristallisation ordonnée de l'eau congelable à l'extérieur des cellules - les cellules se déshydratent, se rétrécissent et l'eau sort de la cellule. Dans le cas contraire, la formation de glace intracellulaire entraîne la destruction des structures membranaires qui est létale pour la cellule.
Pour la plupart des cellules de mammifères, comme cela est le cas pour les produits et médicaments de thérapie cellulaire, que les cellules soient ou non génétiquement modifiées, il est en outre indispensable d'utiliser des cryoprotectants pour préserver l'intégrité et la fonctionnalité cellulaires.

Actuellement, la fabrication à une échelle industrielle (européenne ou mondiale notamment) de produits de thérapie cellulaire pose de nouvelles problématiques, telles que la stabilité du produit fini administré et la variabilité liée à la matière biologique de départ.
Dans la plupart des cas, le produit fini est conditionné :
- frais dans un milieu liquide (type albumine ou solution saline) avec une conservation limitée à quelques heures ou jours, ou bien
- congelé dans une formulation simple à base de DMSO, peu stable et peu efficace à long terme. La quantité non négligeable de cellules mortes, de débris aux effets potentiellement immunogènes et la toxicité pour le patient des excipients couramment utilisés sont autant de limites. La plupart du temps, un lavage des cellules pour retirer ces éléments toxiques (biologiques ou non) est indiqué : ces solutions ne sont pas compatibles avec une distribution industrielle. De plus, elles offrent peu de flexibilité d'administration et de stockage, au contraire des autres classes de médicaments « classiques ».

Il existe donc un besoin pour le développement d'un produit et/ou médicament de thérapie cellulaire qui soit stable à long terme (i.e. pendant plusieurs mois), qui soit facile à utiliser, directement injectable et non toxique.

La présente invention permet de répondre à ce besoin. En effet, le procédé de cryoconservation selon l'invention permet d'obtenir des produits de thérapie cellulaire comprenant des lymphocytes infiltrant la tumeur, prêts à l'emploi (i.e. prêts à être injectés sans lavage, ce qui évite toutes manipulations supplémentaires provoquant une baisse de viabilité et une perte de cellules), stables à long terme, faciles à utiliser et non toxiques.

La présente description divulgue un procédé de cryoconservation d'au moins un échantillon de lymphocytes infiltrant la tumeur, comprenant les étapes suivantes :
i) culture *in vitro* de lymphocytes infiltrant la tumeur à partir de prélèvement de nodules cutanés en transit, ganglion ou métastase issu d'un patient atteint d'un mélanome de stade 3 ou 4, comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés, afin d'obtenir au moins un échantillon de lymphocytes infiltrant la tumeur,
ii) mélange d'au moins un échantillon obtenu à l'étape i) avec une composition comprenant, dans un milieu physiologiquement acceptable :
   a) de la sérum albumine humaine,
   b) au moins un saccharide, et
   c) au moins deux ingrédients choisis parmi le DMSO, la L-cystéine, le coenzyme Q10
   et les alcanediols en C3-C5, puis
iii) congélation du mélange obtenu à l'étape ii).

La présente description divulgue également l'utilisation d'une composition comprenant, dans un milieu physiologiquement acceptable :
a) de la sérum albumine humaine,
b) au moins un saccharide, et
c) au moins deux ingrédients choisis parmi le DMSO, la L-cystéine, le coenzyme Q10 et les alcanediols en C3-C5, pour la cryoconservation d'au moins un échantillon de lymphocytes infiltrant la tumeur (appelés également « TILs » pour « Tumor Infiltrating Lymphocytes »),
ledit échantillon étant obtenu par culture *in vitro* de lymphocytes infiltrant la tumeur à partir de prélèvement de nodules cutanés en transit, ganglion ou métastase d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

Par lymphocytes infiltrant la tumeur (appelés également « TILs » pour « Tumor Infiltrating Lymphocytes »), on entend des lymphocytes infiltrant la tumeur qu'ils soient ou non génétiquement modifiés.

La composition utilisée dans le procédé de cryoconservation décrit (étape ii)) comprend donc, dans un milieu physiologiquement acceptable :
a) de la sérum albumine humaine,
b) au moins un saccharide, et
c) au moins deux ingrédients choisis parmi le DMSO, la L-cystéine, le coenzyme Q10 et les alcanediols en C3-C5.

Par « milieu physiologiquement acceptable », on entend un milieu aqueux comprenant des électrolytes. Les électrolytes sont par exemple des sels de sodium, de potassium, de magnésium et/ou de calcium avec des anions de type chlorure, carbonate, hydroxyde ou caprylate. De préférence, le milieu pharmaceutiquement acceptable est un milieu aqueux comprenant du chlorure de sodium et du caprylate de sodium.

La composition utilisée dans le procédé de cryoconservation décrit comprend la sérum albumine humaine (composé a)). Cette protéine est une protéine plasmatique de haut poids moléculaire (environ 65 kDa). C'est la protéine la plus abondante du plasma ; sa concentration moyenne normale est de 38 à 48 g/l. Elle permet de tamponner le pH et de maintenir l'osmolarité. Sa pureté est de préférence de 95%. On peut également utiliser un ou plusieurs fragments et/ou dérivés de la sérum albumine humaine, lesdits fragments ou dérivés étant non immunogènes et ayant une propriété oncotique similaire à la sérum albumine humaine.
De préférence, la sérum albumine humaine, ses fragments et/ou dérivés, est présente en quantité comprise entre 2 et 10% en poids par rapport au poids total de composition, de préférence entre 2.5 et 6% en poids, de préférence entre 3.5 et 4.5% en poids.
Dans la présente demande, sauf mention contraire, les quantités sont mentionnées en poids par rapport au poids total de composition.

La composition utilisée dans le procédé de cryoconservation décrit comprend également au moins un saccharide (composé b)). Le saccharide améliore la survie et la fonction des cellules en préservant l'équilibre osmotique. Une fraction pénètre les cellules et permet de stabiliser les structures membranaires. Le saccharide est de préférence choisi parmi les monosaccharides, les disaccharides et les trisaccharides.
Les monosaccharides sont de préférence choisis parmi le glucose, le galactose, le fructose et le mannose.
Le disaccharide a de préférence pour formule A-B, dans laquelle A et B sont chacun indépendamment choisis parmi le glucose, le fructose et le mannose. Le saccharide est de préférence un disaccharide. Le disaccharide est de préférence un dimère de glucose. Plus préférentiellement, le disaccharide est choisi parmi le tréhalose et le saccharose. Plus préférentiellement, le disaccharide est le tréhalose.
Les trisaccharides sont de préférence choisis parmi le raffinose (trimère de galactose, glucose et fructose), le maltotriose et l'isomaltotriose (trimères de glucose).
Le saccharide est de préférence présent dans la composition décrite en concentration comprise entre 0.05M et 1M, de préférence entre 0.07M et 0.5M, de préférence entre 0.08M et 0.12M.

La composition utilisée dans le procédé de cryoconservation décrit comprend enfin au moins deux ingrédients choisis parmi le DMSO, la L-cystéine, le coenzyme Q10 et les alcanediols en C3-C5 (composés c)).
Le DMSO, ou diméthylsulfoxyde, est un solvant polaire organique aprotique de formule CH₃-SO-CH₃. C'est un cryoprotectant intracellulaire qui a pour but principal de remplacer le liquide intracellulaire, et qui permet ainsi de prévenir la formation de cristaux de glace et le stress osmotique inhérent aux phases de congélation/décongélation qui font éclater les structures membranaires. Il est de préférence présent dans la composition décrite en quantité comprise entre 2 et 15% en poids, de préférence entre 2.5 et 4.5% en poids.
La L-cystéine est un acide aminé présentant un groupement thiol -SH. Elle est de préférence présente dans la composition en concentration comprise entre 0.05mM et 5mM, de préférence entre 0.5mM et 2mM.
Le coenzyme Q10, également appelé ubiquinone, est un composé chimique comprenant un groupement quinone. Son nom chimique est le 2,3-diméthoxy-5-méthyl-6-décaprénylbenzoquinone. Il est de préférence présent dans la composition en quantité comprise entre 0.005 et 1% en poids, de préférence entre 0.007 et 0.5% en poids, de préférence entre 0.007 et 0.1% en poids.
L'alcanediol en C3-C5 est de préférence un alcane linéaire, ramifié ou cyclique comprenant de 3 à 5 atomes de carbone, et 2 groupements hydroxyl. De préférence, il est choisi parmi les alcanes linéaires comprenant de 3 à 5 atomes de carbone, et 2 groupements hydroxyl. Plus préférentiellement, il est choisi parmi le propane-1,2-diol, le pentane-1,5-diol et le butane-2,3-diol. L'alcanediol en C3-C5 est de préférence le propane-1,2-diol (également appelé propylène glycol).
L'alcanediol en C3-C5 est de préférence présent dans la composition en quantité comprise entre 3 et 10% en volume, de préférence entre 3 et 7% en volume.

De préférence, la composition utilisée dans le procédé de cryoconservation décrit comprend au moins les deux ingrédients suivants comme composés c) :
- du DMSO et de la L-cystéine, ou
- du coenzyme Q10 et un alcanediol en C3-C5, de préférence le propylène glycol.

De préférence, la composition utilisée dans l'étape ii) du procédé de cryoconservation décrit comprend, dans un milieu physiologiquement acceptable :
a) de la sérum albumine humaine, de préférence en quantité comprise entre 2.5 et 6% en poids,
b) un saccharide, de préférence du tréhalose, de préférence en concentration comprise entre 0.07M et 0.5M, et
c) du DMSO et de la L-cystéine, de préférence respectivement en quantité comprise entre 2 et 5% en poids, et en concentration comprise entre 0.5mM et 2mM.

Alternativement, de préférence, la composition utilisée dans l'étape ii) du procédé de cryoconservation décrit comprend, dans un milieu physiologiquement acceptable :
a) de la sérum albumine humaine, de préférence en quantité comprise entre 2.5 et 6% en poids,
b) un saccharide, de préférence du tréhalose, de préférence en concentration comprise entre 0.07M et 0.5M, et
c) du coenzyme Q10 et du propane-1,2-diol, de préférence respectivement en quantité comprise entre 0.007 et 0.1% en poids, et en quantité comprise entre 3 et 10% en volume.

Le procédé de cryoconservation décrit est particulièrement intéressant, et vise à cryoconserver au moins un échantillon de lymphocytes infiltrant la tumeur. En effet, comme démontré en exemple, la composition utilisée dans l'étape ii) du procédé de cryoconservation décrit permet de cryoconserver les TILs, de façon durable et efficace (notamment jusqu'à 4 mois après décongélation).

Les lymphocytes infiltrant la tumeur (ou TILs) sont des lymphocytes typiquement retrouvés dans des nodules cutanés en transit, ganglion ou métastase de patients atteints de mélanome à un stade avancé (stade 3 ou 4). Ils sont impliqués dans la mort des cellules tumorales, et sont actuellement testés dans différents essais cliniques comme produits de thérapie cellulaire. Les TILs proviennent en général de patients eux-mêmes ; ils sont autologues. Pour cela, ils sont prélevés du patient, isolés de la tumeur et sont multipliés *in vitro.*
Plus précisément, l'échantillon de TILs utilisé dans le procédé décrit est obtenu par une étape i) de culture *in vitro* comprenant les étapes suivantes :
- émergence de lymphocytes infiltrant la tumeur contenus dans un prélèvement de nodules cutanés en transit, ganglion ou métastase. Cette émergence est également appelée « culture primaire » ou « sortie » ; c'est l'étape au cours de laquelle les lymphocytes infiltrant la tumeur, initialement contenus dans le prélèvement de nodules cutanés en transit, ganglion ou métastase, passent dans le milieu de culture et sont cultivés *in vitro,*
- stimulation des lymphocytes infiltrant la tumeur issus de l'étape d'émergence, puis enfin
- amplification des lymphocytes infiltrant la tumeur issus de l'étape de stimulation.

L'étape d'émergence des TILs est de préférence réalisée par culture primaire du prélèvement de nodules cutanés en transit, ganglion ou métastase d'un patient atteint d'un mélanome de stade 3 ou 4, de préférence dans un récipient de culture clos. La source de TILs est en effet un échantillon de nodules cutanés en transit, ganglion ou métastase du patient atteint de mélanome de stade 3 ou 4 : elle peut être prélevée par biopsie (prélèvement tumoral). La culture primaire se fait de préférence dans un milieu de culture sélectif sans sérum de type X-VIVO 15® (commercialisé par Cambrex Corp) supplémenté en interleukine-2 (IL-2).
Par « récipient de culture clos », on entend un système permettant d'entretenir des cellules en culture dans un milieu adapté, sans que celles-ci ne soient directement au contact avec l'environnement extérieur. Ainsi, le récipient est clos, i.e. son volume intérieur n'est pas en contact avec l'air ambiant, notamment lors de la culture, lorsque les TILs et le milieu de culture sont introduits dans le récipient.

Après émergence des TILs, ces derniers subissent une étape de stimulation de préférence dans un récipient de culture clos compartimenté. Cela permet une augmentation quantitative significative du rendement global des TILs (jusqu'à 2,5 fois) par comparaison avec la même technique mise en œuvre dans un système ouvert ou non compartimenté. Elle permet également une augmentation qualitative des TILs produits. En effet, parmi les TIL produits, la proportion de TIL CD8+ est supérieure à celle produite (85% versus 60%) en condition classique, et présente une activité cytotoxique spécifique vis-à-vis de la lignée tumorale autologue plus importante (14% versus 2%).
Selon une mise en œuvre préférée, les TILs sont stimulés en présence au moins de cellules nourricières allogéniques irradiées non proliférantes. Ces cellules nourricières allogéniques sont rendues non proliférantes par exemple par irradiation. De préférence, on stimule les TILs par co-culture avec un mélange formé de cellules mononucléées issues de sang de donneur humain sain (non proliférantes) et de cellules B transformées par le virus d'Epstein Barr (non proliférantes), de préférence la lignée LAZ388.
De préférence, on co-cultive les cellules nourricières allogéniques non proliférantes et les TILs dans un milieu de culture sélectif sans sérum de type X-VIVO 15® (commercialisé par Cambrex Corp) supplémenté en interleukine-2 (IL-2). En outre, au cours de l'étape de stimulation, on introduit de préférence dans le récipient de stimulation clos, parallèlement aux lymphocytes, un agent mitotique tel que la PHA-L (phytohémagglutinine de type L) favorable à l'amplification des lymphocytes.
Généralement, le récipient de culture clos compartimenté comporte au moins deux compartiments, dits l'un chambre technique, l'autre chambre de tranquillisation, communiquant entre eux, et au moins une voie d'accès débouchant dans l'un des compartiments. On introduit les TILs et les cellules nourricières allogéniques irradiées non proliférantes en suspension dans un milieu de culture par l'intermédiaire de ladite voie d'accès, et on renouvelle le milieu de culture par l'intermédiaire de la ou d'une autre voie d'accès.

L'étape de stimulation des TILs est suivie d'une étape d'amplification. Typiquement, les TILs stimulés sont récoltés puis transférés en système de culture. Les poches sont incubées, et 2 fois par semaine, du milieu neuf additionné d'IL-2 est ajouté à chaque poche.
On obtient ainsi des poches dont la concentration en TILs est comprise entre 1.10⁶ et 3. 10⁶ lymphocytes/ml.

II est possible de mettre en œuvre les étapes d'émergence, de stimulation et d'amplification dans un récipient de culture clos différent d'une étape à une autre. Dans ce cas, le transfert d'un récipient clos à un autre est réalisé de façon stérile, par exemple en connectant, à l'aide d'un appareil de connexion stérile de type SCD® de Terumo, les tubulures servant de voies d'accès. En variante, les récipients clos d'émergence, de stimulation et d'amplification sont pré-connectés entre eux au moment de la fabrication, de sorte à former un système clos.

Un tel système permet de réaliser la culture complète des lymphocytes T en système clos, limitant ainsi le risque de contamination.

Dans le procédé de cryoconservation décrit, l'étape de mélange ii) de l'échantillon de TILs avec la composition décrite ci-dessus se fait typiquement par dilution. De préférence, l'échantillon de TILs est repris dans le composé a) sous forme liquide, de préférence pour 50% du volume, puis les composés b) et c), de préférence précédemment mélangés entre eux pour obtenir une solution de concentration 2x, sont ajoutés. Le mélange de l'étape i) peut se faire aux alentours de 4°C, ou à température ambiante (i.e. 20°C).
De préférence, l'échantillon de TILs subit une centrifugation, le surnageant est retiré et le culot est suspendu dans la composition décrite ci-dessus.

Quant à la congélation (étape iii)) du procédé de cryoconservation décrit, elle est de préférence effectuée sur une descente en température de +4°C ou température ambiante jusqu'à une température comprise entre -100°C et -160°C. L'étape de congélation (étape ii)) est de préférence effectuée jusqu'à une température comprise entre -100°C et -180°C, de préférence comprise entre -140°C et -160°C.
L'échantillon est ensuite stocké à une température inférieure à -130°C en général.
De préférence, la congélation iii) est réalisée en plaçant le mélange obtenu dans l'étape ii) dans un récipient immergé dans un mélange d'isopropyl alcool à +4°C, le tout étant mis à une température comprise entre -70°C et -100°C. Ce système (« congélation en boîte Nalgène ») permet, grâce au refroidissement lent de l'alcool, une descente en température quasi-linéaire comprise entre -1°C et -2°C par minute. Alternativement, de préférence, la congélation iii) est effectuée à l'aide d'un congélateur programmé. De tels congélateurs sont notamment commercialisés par Air Liquide ou Cryobiosystem. Ils permettent notamment une descente en température plus reproductible et des schémas plus complexes.
De préférence, la congélation iii) se fait, notamment à l'aide d'un congélateur programmé, selon les étapes suivantes :
- placer le mélange obtenu à l'étape ii) à une température de +4°C ; puis
- diminuer la température de 1°C par minute, de +4°C à -40°C ; puis
- diminuer la température de 10°C par minute, de -40°C à -150°C, pour atteindre une température finale de stockage d'environ -150°C en moyenne, en conteneur d'azote liquide ou gazeux.

Le produit ainsi obtenu, congelé, peut être conservé pendant quelques mois à -150°C environ.

L'invention va maintenant être exemplifiée à l'aide des exemples qui suivent, qui ne sont pas limitatifs.

### Exemple 1: préparation et cryoconservation d'un échantillon de TILs selon le procédé de l'invention

Le produit TILs constitue un Médicament de Thérapie Innovante (MTI) de nature autologue. Il est constitué de cellules vivantes exclusivement destinées au patient, donneur de la matière de départ (en l'occurrence le ganglion prélevé sur le patient). Les TILs d'un patient, après amplification *ex vivo,* seront réinjectées à ce même patient : c'est un système autologue.

### Le procédé est le suivant :

La matière première de départ est constituée de lymphocytes infiltrant les tumeurs métastatiques isolées de ganglions de patients atteints de mélanome malin cutané.

Les patients subissent préalablement un criblage virologique (CMV, hépatites B et C, HIV, HTLV, EBV et parvovirus B19).

### Etape i) du procédé : obtention d'un échantillon de TILs :

Le(s) morceau(x) de prélèvements (cutanés, pulmonaires et/ou ganglionnaires) envahis de cellules de mélanome au stade métastatique sont recueillis aseptiquement en per-opératoire, puis placés « à sec » (sans conditionnement dans un milieu de transport) dans des contenants stériles.

Ils sont ensuite découpés en fragments de 1 à 2 mm. Les fragments de chaque prélèvement tumoral sont ensuite congelés en cryotube, dans l'attente de leur préparation.

### Etape d'émergence de TILs :

Les cryotubes de fragments tumoraux sont décongelés et placés dans des puits de plaques de culture 12 puits contenant du milieu de culture X VIVO 15® et de l'IL-2. Les plaques sont incubées à 37°C, à 5% de CO2.

Les puits sont examinés au microscope inversé et le milieu de culture additionné d'IL-2 est renouvelé par moitié du volume 2 fois par semaine.

Au bout de 10-12 jours de culture, la totalité des TILs en sortie des morceaux tumoraux est récoltée, et une quantité définie de ces TILs est utilisée pour stimulation.

### Etape de stimulation de TILs :

Les TILs viables obtenus précédemment sont mis en co-culture, en présence de PHA-L, avec des cellules nourricières irradiées composées de lymphocytes allogéniques (issus d'une banque sécurisée de cellules mononucléées constituée à partir de résidus d'anneau de kit de don de CPA de 3 donneurs sains) et de cellules LAZ irradiées (lignée B allogénique transformée par le virus d'Epstein Barr : lignée LAZ388).

L'irradiation des cellules nourricières est nécessaire pour bloquer la prolifération de ces cellules sans nuire à leurs effets nourriciers (production de facteurs solubles, expression de marqueurs à leur surface...) importants dans l'activation et la prolifération des TILs.

TILs (1,8.10⁶ cellules) et cellules nourricières (lymphocytes allogéniques : 240.10⁶ cellules; LAZ388 : 120.10⁶ cellules) sont mis en culture dans 60 plaques 96 puits dans un milieu de culture X VIVO 15® et de l'IL-2. Les plaques sont incubées à 37°C, à 5% de CO2.

2 fois par semaine, le tiers supérieur du milieu présent dans les puits est éliminé et remplacé par du milieu neuf et de l'IL-2. Après 10 à 13 jours, les TILs ont proliféré et forment une pastille opaque au fond des puits. Le contenu de tous les puits est alors recueilli.

### Etape d'amplification de TILs :

Les TILs récoltés à partir des plaques 96 puits sont transférés en poche de culture et la concentration cellulaire réajustée. Les poches sont incubées et 2 fois par semaine, du milieu neuf X VIVO 15® et de l'IL-2 sont ajoutés à chaque poche, de manière à ajuster la concentration des TILs dans les poches à 1.10⁶ lymophocytes/ml.

### Etape ii) du procédé : mélange de l'échantillon de TILs avec la composition de cryoconservation :

Après 10 jours de culture en poche (étape d'amplification), le volume de suspension cellulaire récolté à partir des poches de TILs est réduit par centrifugation pour concentrer les TILs, qui sont lavés en eau physiologique tamponnée (NaCl 0.9%). Le culot contenant les cellules est ensuite mélangé avec l'une des compositions suivantes :
- la « composition A », comprenant 3.5% de DMSO, 1mM de L-cystéine, 0.1M de tréhalose et 4% de sérum albumine humaine (AH) ; ou
- la « composition B », comprenant 5% de propylène glycol, 0.01% de coenzyme Q10, 0.1M de tréhalose et 4% de sérum albumine humaine (AH).

### Etape iii) du procédé : congélation du mélange obtenu :

Chaque mélange obtenu à l'issue de l'étape ii) est congelé à -150°C.

Chaque produit est conservé sous forme congelée en conteneur d'azote gazeux à une température inférieure à -150°C, en attendant la décongélation et l'administration au patient.

Chaque cryotube réalisé contient :
1. 75x10⁶ cellules viables
2. Une concentration cellulaire constante fixée à 75x10⁶ cellules viables/mL.
3. Le volume du produit est de 1 ml
4. Sa composition cellulaire est la suivante :
   a. Substance active :
      i. Plus de 99% de cellules CD45⁺.
      ii. Plus de 50% de lymphocytes T CD45⁺ CD3⁺ CD8⁺ immunocompétents co-exprimant l'IFNg et CD107a en réponse à une activation CD3⁺ CD25⁺ (= test de potentialité).
   b. Impuretés :
      i. Traces de cellules tumorales HMB45⁺ (< 1% limite de détection de la méthode d'analyse utilisée).
      ii. Traces de cellules lymphoïdes B CD45⁺ CD3⁻ CD19⁺ (< 1% limite de détection de la méthode d'analyse utilisée).
      iii. Traces de cellules NK CD45⁺ CD3⁻ CD56⁺ et/ou CD16⁺ (< 1% limite de détection de la méthode d'analyse utilisée).
      iv. Débris cellulaires (= détection par cytométrie en flux ou par dosage de l'ADN résiduel).

### Exemple 2: efficacité des compositions dans la cryoconservation de TILs selon l'invention

Les cellules utilisées ici sont des TILs obtenus comme décrit à l'étape i) de l'exemple 1.

### La formulation :

Les compositions selon l'invention sont testées en comparaison à :
- une formulation simple (témoin négatif), connue et largement utilisée dans les unités de transplantation de cellules hématopoïétiques, mais à la capacité de conservation limitée : c'est un mélange de DMSO 10% + sérum albumine humaine (AH) 4% ; et
- une formulation fraîche où les cellules sont placées en sérum albumine humaine 4% et analysées directement, qui est le témoin positif.

### 2 compositions selon l'invention sont évaluées :

- la « composition A », comprenant 3.5% de DMSO, 1mM de L-cystéine, 0.1M de tréhalose et 4% de sérum albumine humaine (AH) ; et
- la « composition B », comprenant 10% de propylène glycol, 0.01% de coenzyme Q10, 0.1M de tréhalose et 4% de sérum albumine humaine (AH).

### Le mode de congélation :

- la congélation en boîte Nalgène est la référence manuelle connue : les tubes sont placés dans une boîte hermétique remplie avec un mélange d'isopropane-alcool à +4°C, puis l'ensemble est placé à -80°C et le refroidissement lent de l'alcool permet une descente en température quasi-linéaire comprise entre -1°C et -2°C par minute ;
- la congélation automatisée à l'aide du CRF (congélateur programmé) est commandée électroniquement, elle permet la réalisation de courbes à façon linéaires ou non linéaires pour optimiser les rendements de congélation. Cette méthode est reproductible.

Les cellules sont évaluées en point final après décongélation suite à 1 mois de stockage sous forme congelée à -150°C maximum.

### Matériels & Méthodes

### Cryoprotectants et réactifs

| **Matériel** | **Fabricant** |
|---|---|
| Propylène glycol | NA |
| Tréhalose dihydrate | NA |
| Albumine humaine | LFB |
| DMSO | Wak-chemie ou Miltenyi |
| Coenzyme Q10 | NA |
| | |
| CFSE (carboxyfluorescein diacetate succinimidyl ester, marqueur intracellulaire fluorescent) | Life technologies |
| anticorps utilisés | |
| Billes CD3/CD28 | |

### Appareils

| **Matériel** | **Fournisseur** |
|---|---|
| CRF (congélateur programmé) | • Cryobiosystem (gamme-digit cool) |
| | • Air Liquide (gamme freezal) |
| Cytomètre en flux | NA |
| Compteur de cellules | Type Nucléocounter NC200 (Chemometec) |

### Méthodes

A partir d'une seule matière de départ qui est une suspension cellulaire fraîche, issue d'une culture continue de cellules / d'un procédé normal, 4 bras d'études sont envisagés :
- **Bras T0 commun** = Il s'agit des cellules fraîches T0 analysées avant centrifugation en milieu de culture.
- **Bras référent (Témoin négatif)** (tubes 1) = il s'agit de cellules congelées en DMSO 10% + AH 4%, selon une méthode par palier grâce à une boîte Nalgène (méthode manuelle). Cette méthode est décrite comme modérément stressante pour les cellules lors d'une étude antérieure en comparaison à des cellules témoins positifs non congelées.
- **Bras Témoin positif T1** = il s'agit de cellules non congelées maintenues en sérum albumine humaine 4% pour simuler une formulation fraîche injectable.
- **Bras test** (tubes 2) = il s'agit de cellules congelées dans une composition selon l'invention (composition A ou B), par la méthode par congélation en CRF (tubes 3).

De façon plus précise, le protocole est le suivant :
∘ Les cellules fraîches du T0 sont dans les conditions de culture appropriées définies par l'UTCG de Nantes. Enumérer les cellules TILs, mesurer leur viabilité et leur prolifération (700.10⁶ cellules vivantes sont nécessaires pour toutes les conditions) : cela établit le T0 = référence cellules fraîches non formulées
∘ Cette expérience a été réalisée 3 fois de manière indépendante (sur 3 produits finis différents provenant de 3 donneurs différents)
∘ Réaliser 3 cryotubes de 75.10⁶ cellules pour chaque condition
∘ Le T0 et T1 sont réalisés 1 seule fois (10⁷ cellules par condition)
∘ Répartir la suspension dans 1 flacon de 250mL, soit 225.10⁶ cellules vivantes par tube
∘ Centrifuger à 400g pendant 8 minutes
∘ Chaque cryotube a un volume de suspension final de 1mL et une concentration cellulaire de 75x10⁶ cellules vivantes/ml
∘ Après centrifugation, retirer le surnageant avec précaution, à l'aide d'une micropipette ou d'une pompe aspirante
∘ Reprendre les cellules dans 1,5mL d'AH 4% pour les conditions des tubes 1 et 2
∘ Transférer la suspension cellulaire (= 500µL) dans les 3 cryotubes numérotés
∘ Ajouter 500µL du milieu de congélation à une concentration 2x au goutte à goutte
∘ Refermer les cryotubes
∘ Les tubes 2 sont placés dans une composition selon l'invention puis sont mis à incuber 10 minutes à +4°C pour la formulation à base de DMSO et 30 minutes à +4°C pour la formulation à base d'alcanediols en C3-C5 puis congelés au CRF.
∘ Les tubes 1 sont congelés en boîte Nalgène précédemment stabilisée à +4°C.

Les cryotubes du bras test sont congelés grâce à un « Controlled Rate Freezer » (CRF) où ils subissent une descente en température programmée, après une incubation préalable à +4°C, de -1°C par minute jusqu'à un palier de -40°C puis une congélation rapide de -10°C/minute jusqu'à la température de stockage (-130°C). Le stockage se fait ensuite en conteneur d'azote gazeux à -150°C.

Les cryotubes du bras référent sont placés dans une boîte Nalgène (= freezing box) placée à +4°C. Le tout est transféré directement au congélateur -80°C durant la nuit. Les cryotubes sont transférés en conteneur d'azote gazeux pour le stockage après 48 heures au plus tard.

### A décongélation :

Après 1 mois minimum, les cryotubes sont décongelés par une méthode rapide. Pour cela, le cryotube est sorti du conteneur à azote puis transporté jusqu'aux locaux Contrôle Qualité grâce à une boîte Nalgène à -80°C. A l'arrivée, il est immédiatement plongé dans un bain marie à +37°C jusqu'à disparition complète des glaçons (après environ 1 minute). Le tout est directement numéré et analysé selon les tests décrits dans le paragraphe « critères d'évaluation » ci-dessous. La même analyse est effectuée 4 heures plus tard pour le T1 Témoin positif formulé en sérum albumine humaine.

### Nomenclature des cryotubes :

| **Milieu** | **Cellules T0 fraîches** | **Milieu 1 (témoin négatif-congélation DMSO 10% Nalgène)** | **Milieu 2 (témoin positif T1)** | **Milieu 3 (DMSO)** | **Milieu 4 (PG)** |
|---|---|---|---|---|---|
| **N° des ampoules** | NA | 1-A à C | NA | 2-A à C | 2'-A à C |

| | | | | | |
|---|---|---|---|---|---|
| *NA : Non Applicable.* *Concentration cellulaire : La concentration cellulaire est identique pour toutes les ampoules soit 75.10⁶ cellules dans un millilitre et par ampoule.* | | | | | |

### Critères d'évaluation

Les critères d'évaluation sont :
- Viabilité par méthode automatique à décongélation et après 4 heures (type nucléocounter).
- Numération des cellules vivantes à décongélation et après 4 heures.
- Test de prolifération des cellules sur 3 jours (maintien sur 2 jours après remise en culture : par test CFSE).

Les cellules sont analysées après décongélation rapide dans un bain-marie thermostaté à +37°C. La décongélation est rapide jusqu'à disparition totale des glaçons (simule la préparation d'un produit injectable à l'homme) puis le tube est conservé à température ambiante (15°C-25°C) durant 4 heures pour vérifier la stabilité décongelée.

### Résultats

Les résultats figurent dans les Tableaux 1 à 3 ci-dessous.

Pour qu'une composition soit retenue pour les TILs, elle doit donner un résultat supérieur à 85% du témoin positif (T1: cellules formulées en sérum albumine, AH, 4%) et significativement supérieur au témoin négatif (DMSO 10% + AH4%) à décongélation, et la baisse ne doit pas excéder 20% du Témoin positif après 4 heures.
Dans le cas du rapport CD8/CD4 qui documente la stabilité des populations cellulaires dans le produit fini, le rapport ne doit pas varier de plus de 40%.

**Tableau 1 : viabilité post-décongélation en AH4% déterminée par comptage manuel ou éosine.**

| | | **T0 (référence)** | **T1 (Témoin positif)** | **Composition A** | **Composition B** | **Témoin négatif** |
|---|---|---|---|---|---|---|
| **Lot 1** | **Viabilité en %** | 86,80 | 79,90 | 81,60 | 85,00 | 72,10 |
| | **taux de recouvrement T0** | 100,00 | 92,05 | 94,01 | 97,93 | 83,06 |
| | **Viabilité en % du T0** | 0,0 | -7,9 | -6,0 | -2,1 | -16,9 |
| **Lot 2** | **Viabilité en %** | 85,90 | 74,11 | 73,40 | 77,30 | 56,30 |
| | **taux de recouvrement T0** | 100,00 | 86,27 | 85,45 | 89,99 | 65,54 |
| | **Viabilité en % du T0** | 0,0 | -13,7 | -14,6 | -10,0 | -34,5 |
| **Lot 3** | **Viabilité en %** | 85,88 | 82,90 | 75,50 | 80,70 | 67,80 |
| | **taux de recouvrement T0** | 100,00 | 96,53 | 87,91 | 93,97 | 78,95 |
| | **Viabilité en % du T0** | 0,0 | -3,5 | -12,1 | -6,0 | -21,1 |
| **moyennes** | **Viabilité en %** | 86,19 | 78,97 | 76,83 | 81,00 | 65,40 |
| | **Viabilité en % du T0** | 0,0 | -8,4 | -10,9 | -6,0 | -24,1 |
| | **Taux de recouvrement** | 100,0 | 91,6 | 89,1 | 94,0 | 75,9 |
| | **T0** | | | | | |
| | **Ecart type recovery rate** | 0,0 | 5,1 | 4,4 | 4,0 | 9,2 |
| | **taux de recouvrement T1** | 109,1 | 100,0 | 97,3 | 102,6 | 82,8 |
| | **Ecart type (moyenne)** | 0,5 | 4,5 | 4,3 | 3,9 | 8,2 |
| | **SEM** | 0,3 | 2,6 | 2,5 | 2,2 | 4,7 |

Après 4h à température ambiante (TA), la viabilité moyenne obtenue avec la composition A est de 69.20% (87,6% du Tl), et avec la composition B elle est de 70.97% (89,9% du Tl), ce qui est significativement supérieur à la viabilité du témoin négatif (53,00%).

| | | **T0** | **T1** | **Composition A** | **Composition B** | **Témoin négatif** |
|---|---|---|---|---|---|---|
| **Lot 1** | **cellules proliférantes en %** | 64,80 % | 60,12% | 75,49% | 83,70% | 66,19% |
| | **taux de recouvrement T0** | 100,0 0% | 92,78% | 116,50% | 129,17% | 102,15 % |
| | **en % du T0** | 0,0 | -38,2% | -22,4% | -14,0% | -32,0% |
| **Lot 2** | **cellules proliférantes en %** | 96,54 % | 96,48% | 93,53% | 90,89% | 94.02% |
| | **taux de recouvrement T0** | 100,0 0% | 99,94% | 96,88% | 94,15% | 97,39% |
| | **en % du T0** | 0,0 | -0,9% | -3,9% | -6,6% | -3,4% |
| **Lot 3** | **cellules proliférantes en %** | 98,34 % | 97,92% | 92,53% | 91,76% | 59,00% |
| | **taux de recouvrement T0** | 100,0 0% | 99,57% | 94,09% | 93,31% | 60,00% |
| | **en % du T0** | 0,0% | 11,0% | 4,9% | 4,0% | -33,1% |
| **Moyennes** | **cellules proliférantes en %** | 81,57 % | 79,02% | 87,18% | 88,78% | 73,07% |
| | **en % du T0** | 0,0% | -3,13 | 6,88 | 8,84 | -10,42 |
| | **en % du T1** | 3,23 | 0,00 | 10,33 | 12,36 | -7,53 |
| | Ecart type | 18,87 % | 21,42% | 10,14% | 4,42% | 18,50% |
| | SEM | 10,89 % | 12,37% | 5,85% | 2,55% | 10,68% |
| | **Taux de recouvrement T1** | | **100,00 %** | **110,33%** | **112,36%** | **92,47%** |
| | **Taux de recouvrement T0** | 100,0 % | 96,9% | 106,9% | 108,8% | 89,6% |
| | **Ecart type taux de recouvrement** | 0,0% | 4,0% | 12,2% | 20,5% | 23,1% |

**Tableau 2 : prolifération des TILs sur 2 jours après marquage CFSE.**

| Lot 1 | T0 | **T1** | **Composition A** | **Composition B** | **Témoin négatif** |
|---|---|---|---|---|---|
| CD45+ | | | | | |
| CD45+CD3+CD4+ | 66,68% | 67,18% | **60,90%** | **61,00%** | **60,70%** |
| CD45+CD3+CD8+ | 28,72% | 28,33% | **30,50%** | **30,90%** | **31,20%** |
| Contrôle | | | OK | OK | OK |
| CD45+CD3+CD25+ | 14,49% | 12,55% | **14,90%** | **15,30%** | **14,50%** |
| rapport CD8/CD4 | 0,43 | 0,42 | **0,50** | **0,51** | **0,51** |
| | | | | | |

| Lot 2 | T0 | **T1** | **Composition A** | **Composition B** | **Témoin négatif** |
|---|---|---|---|---|---|
| CD45+ | 99,86% | 99,44% | 99,92% | 99,60% | 99,09% |
| CD45+CD3+CD4+ | 47,30% | 46,65% | **56,30%** | **54,33%** | **55,89%** |
| CD45+CD3+CD8+ | 48,26% | 47,63% | 20,12% | 20,07% | 21,33% |
| Contrôle | 0,56% | 0,39% | 0,74% | 0,52% | 0,57% |
| CD45+CD3+CD25+ | 16,47% | 12,91% | **50,82%** | **47,69%** | **38,48%** |
| rapport CD8/CD4 | 1,02 | 1,02 | 0,36 | 0,37 | 0,38 |
| | | | | | |

| Lot 3 | T0 | **T1** | **Composition A** | **Composition B** | **Témoin négatif** |
|---|---|---|---|---|---|
| CD45+ | 99,88% | 99,88% | 99,87% | 99,85% | 99,74% |
| CD45+CD3+CD4+ | 77,14% | 77,46% | **76,11%** | **76,70%** | **70,24%** |
| CD45+CD3+CD8+ | 16,70% | 16,98% | **17,09%** | **16,58%** | **19,25%** |
| Contrôle | 0,38% | 0,38% | 0,54% | 0,50% | 0,46% |
| CD45+CD3+CD25+ | 35,95% | 30,81% | **43,78%** | **43,16%** | **40,11%** |
| rapport CD8/CD4 | 0,22 | 0,22 | **0,22** | **0,22** | 0,27 |
| | | | | | |

| Moyennes | T0 | **T1** | **Composition A** | **Composition B** | **Témoin négatif** |
|---|---|---|---|---|---|
| CD45+CD3+CD4+ | 63,71% | 63,76% | **64,44%** | **64,01%** | **62,28%** |
| CD45+CD3+CD8+ | 31,23% | 30,98% | 22,57% | 22,52% | 23,93% |
| CD45+CD3+CD25+ | 22,30% | 18,76% | **36,50%** | **35,38%** | **31,03%** |
| *CD25+* | *11,86%* | *10,44%* | *19,03%* | *17,54%* | *14,34%* |
| tx de recouvrement T1 | 118.9% | 100% | **194.6%** | **188,6%** | **165.4%** |
| écart type Tx de recouvrement | 0,0% | 4,5% | 113,7% | 102,3% | 74,0% |
| rapport CD8/CD4 | 0,56 | 0,55 | 0,36 | 0,36 | 0,39 |

**Tableaux 3 : caractérisation des cellules par cytométrie en flux.**

| | | **T0** | **T1** | **Composition A** | **Composition B** | **Témoin négatif** |
|---|---|---|---|---|---|---|
| **lot 1** | **% de cellules IFNg⁺/CD107⁺ parmi CD8⁺** | 66,6 | 71,96 | **73,0** | **66,5** | **64,0** |
| | **tx de recouvrement T1** | 92,6 | 100,0 | 101,4 | 92,5 | 88,9 |
| **lot 2** | **% de cellules IFNg⁺/CD107⁺ parmi CD8⁺** | 77,0 | 83,68 | **63,1** | **64,7** | **53,1** |
| | **tx de recouvrement T1** | 92,0 | 100,0 | 75,4 | 77,3 | 63,4 |
| **lot 3** | **% de cellules IFNg⁺/CD107⁺ parmi CD8⁺** | 85,3 | 85,9 | **83,7** | **78,8** | **62,7** |
| | **tx de recouvrement T1** | 99,3 | 100,0 | 97,4 | 91,8 | 73,0 |
| **Moyennes** | **% de cellules IFNg⁺/CD107⁺ parmi CD8⁺** | 76,3 | 80,5 | **73,2** | **70,0** | **59,9** |
| | **Tx de recouvrement (T1)** | 94,8% | 100,0% | **91,0%** | **87,0%** | **74,4%** |
| | **Ecart type** | 4,1 | 0,0 | 14,0 | 8,6 | 12,9 |

En conclusion, les 2 formulations testées atteignent les spécifications fixées (moins de 15% d'écart par rapport au témoin positif : des cellules fraîches formulées en sérum albumine 4% qui est une solution injectable).

### Pour la formulation A :

- La viabilité atteint 97.3% de la valeur du témoin positif à décongélation et se maintient à 87,6% après 4 heures (76,8% à décongélation puis 69,2% après 4 heures en valeur absolue). Cela confirme que la formulation est efficace et que la quantité de débris de cellules mortes est limitée.
- La capacité de prolifération des cellules après 2 jours de remise en culture post-décongélation est supérieure au témoin positif (+10,3%) : ce qui valide leur capacité de reprise/persistance après décongélation.
- La fonctionnalité des cellules est préservée puisque leur capacité de dégranulation (= synthèse de cytokines médiatrices de leur activité immune, en réponse à une activation CD3/CD28) est préservée (91,0% du Témoin positif en taux de recouvrement).
- Le pourcentage de cellules activées CD25+ (partie du récepteur à l'IL-2) est supérieur au témoin positif (+ 94,6%) et reflète également les capacités d'activation/prolifération des cellules.
- Seul le ratio CD8/CD4 est altéré (0,36 au lieu de 0,55) du fait de la baisse de la proportion de lymphocytes CD8⁺ cytotoxiques connus pour être plus sensibles aux cycles de congélation/décongélation. Cette baisse modérée est tolérée pour le produit.

### Pour la formulation B :

- La viabilité atteint 102.6% du témoin positif et se maintient à 89,9% après 4 heures (soit .81% de cellules viables à décongélation et 71% après 4 heures).
- La capacité de prolifération des cellules sur 2 jours après décongélation est supérieure au témoin positif (112,4% en taux de recouvrement).
- La fonctionnalité des cellules est préservée puisque la capacité de dégranulation (synthèse de cytokines en réponse à une activation CD3/CD28) est de 87,0%.
- Le pourcentage de cellules activées est supérieur au témoin positif (+ 88,6%).
- Seul le ratio CD8/CD4 est altéré (0,36 au lieu de 0,55) du fait d'une mortalité accrue des lymphocytes CD8⁺ cytotoxiques qui n'altère pas la fonctionnalité des cellules.

### Conclusion :

Cette étude met en évidence que les formulations A et B :
- sont supérieures au Témoin négatif DMSO 10% + AH 4% réalisé en boîte Nalgène, dont l'efficacité de congélation et la stabilité sont limitées ; et
- sont similaires aux spécifications du témoin positif non congelé (moins de 15% d'écart par rapport aux cellules fraîches en albumine humaine 4%).

Cela démontre que l'utilisation de formulations complexes aux composés synergiques ont permis d'améliorer significativement les caractéristiques générales des cellules à décongélation.

L'intérêt de ces formulations réside dans les propriétés suivantes :
- non toxicité c'est-à-dire prête à l'utilisation après décongélation, ce qui permet de s'affranchir de toute manipulation pharmaceutique à l'hôpital,
- stabilité congelée de longue durée des cellules (plusieurs mois),
- stabilité décongelée de 4 heures englobant le temps de préparation et de manipulation avant injection au patient,
- compatible avec les contraintes industrielles de stockage, et
- préservation des caractéristiques générales et fonctionnelles des cellules.

Elles répondent ainsi aux problématiques courantes rencontrées en thérapie cellulaire.

## Revendications

1. Procédé de cryoconservation d'au moins un échantillon de lymphocytes infiltrant la tumeur, comprenant les étapes suivantes :
i) culture *in vitro* de lymphocytes infiltrant la tumeur à partir de prélèvement de nodules cutanés en transit, ganglion ou métastase issu d'un patient atteint d'un mélanome de stade 3 ou 4, comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés, afin d'obtenir au moins un échantillon de lymphocytes infiltrant la tumeur,
ii) mélange d'au moins un échantillon obtenu à l'étape i) avec une composition comprenant, dans un milieu physiologiquement acceptable :
a) de la sérum albumine humaine en quantité comprise entre 2 et 10% en poids,
b) au moins un saccharide en concentration comprise entre 0.05M et 1M, et
c) du DMSO et de la L-cystéine, respectivement en quantité comprise entre 2 et 15% en poids, et en concentration comprise entre 0.05mM et 5mM, ou
du coenzyme Q10 et du propane-1,2-diol, respectivement en quantité comprise entre 0.005 et 1% en poids, et en quantité comprise entre 3 et 10% en volume, puis
iii) congélation du mélange obtenu à l'étape ii).

2. Le procédé de cryoconservation selon la revendication 1, **caractérisée en ce que** le saccharide est choisi parmi les monosaccharides, les disaccharides et les trisaccharides.

3. Le procédé de cryoconservation selon la revendication 2, **caractérisée en ce que** le saccharide est un disaccharide choisi parmi le tréhalose et le saccharose.

4. Le procédé de cryoconservation selon l'une des revendications 1 à 3, **caractérisée en ce que** la sérum albumine humaine est présente en quantité comprise entre 2.5 et 6% en poids.

5. Le procédé de cryoconservation selon l'une des revendications 1 à 4, **caractérisée en ce que** le saccharide est présent en concentration comprise entre 0.07M et 0.5M.

6. Le procédé de cryoconservation selon l'une des revendications 1 à 5, **caractérisée en ce que** la composition utilisée à l'étape ii) comprend :
a) de la sérum albumine humaine, en quantité comprise entre 2.5 et 6% en poids,
b) du tréhalose, en concentration comprise entre 0.07M et 0.5M, et
c) du DMSO et de la L-cystéine, respectivement en quantité comprise entre 2 et 15% en poids, et en concentration comprise entre 0.5mM et 2mM.

7. Le procédé de cryoconservation selon l'une des revendications 1 à 5, **caractérisée en ce que** la composition utilisée à l'étape ii) comprend :
a) de la sérum albumine humaine, en quantité comprise entre 2.5 et 6% en poids,
b) du tréhalose, en concentration comprise entre 0.07M et 0.5M, et
c) du coenzyme Q10 et du propane-1,2-diol, respectivement en quantité comprise entre 0.007 et 0.1% en poids, et en quantité comprise entre 3 et 10% en volume.

8. Le procédé de cryoconservation selon l'une des revendications 1 à 7, **caractérisé en ce que** la congélation iii) est effectuée jusqu'à une température comprise entre -100°C et -180°C, de préférence comprise entre -140°C et -160°C.

9. Le procédé de cryoconservation selon l'une des revendications 1 à 8, **caractérisé en ce que** la congélation iii) est réalisée en plaçant le mélange obtenu en ii) dans un récipient immergé dans un mélange d'isopropyl alcool à +4°C, le tout étant mis à une température comprise entre -70°C et -100°C.

10. Le procédé de cryoconservation selon l'une des revendications 1 à 9, **caractérisé en ce que** la congélation iii) est effectuée à l'aide d'un congélateur programmé.

11. Utilisation d'une composition comprenant, dans un milieu physiologiquement acceptable :
a) de la sérum albumine humaine en quantité comprise entre 2 et 10% en poids,
b) au moins un saccharide en concentration comprise entre 0.05M et 1M, et
c) du DMSO et de la L-cystéine, respectivement en quantité comprise entre 2 et 15% en poids, et en concentration comprise entre 0.05mM et 5mM, ou
du coenzyme Q10 et du propane-1,2-diol, respectivement en quantité comprise entre 0.005 et 1% en poids, et en quantité comprise entre 3 et 10% en volume, pour la cryoconservation d'au moins un échantillon de lymphocytes infiltrant la tumeur,
ledit échantillon étant obtenu par culture *in vitro* de lymphocytes infiltrant la tumeur à partir de prélèvement de nodules cutanés en transit, ganglion ou métastase issu d'un patient atteint d'un mélanome de stade 3 ou 4, ladite culture comprenant l'émergence desdits lymphocytes infiltrant la tumeur contenus dans le prélèvement de nodules cutanés en transit, ganglion ou métastase, puis la stimulation des lymphocytes infiltrant la tumeur issus de l'étape d'émergence, puis enfin l'amplification des lymphocytes infiltrant la tumeur stimulés.

12. Le procédé de cryoconservation selon l'une des revendications 1 à 10, ou l'utilisation selon la revendication 11, dans lequel la stimulation des lymphocytes infiltrant la tumeur issus de l'étape d'émergence se fait en présence au moins de cellules nourricières allogéniques irradiées non proliférantes.

13. Le procédé de cryoconservation selon l'une des revendications 1 à 10 ou 12, ou l'utilisation selon la revendication 11 ou 12, dans lequel la stimulation des lymphocytes infiltrant la tumeur issus de l'étape d'émergence se fait par co-culture avec un mélange formé de cellules mononucléées issues de sang de donneur humain sain et de cellules B transformées par le virus d'Epstein Barr, de préférence la lignée LAZ388, toutes non proliférantes.

## Patentansprüche

1. Verfahren zur Kryokonservierung von mindestens einer Probe von Lymphozyten, die den Tumor infiltrieren, umfassend die folgenden Schritte:
i) *In vitro* Kultur von Lymphozyten, die den Tumor infiltrieren, aus einer Entnahme von Hautknoten im Transit, einem Lymphknoten oder einer Metastase eines Patienten, der an einem Melanom im Stadium 3 oder 4 leidet, umfassend die Emergenz der Lymphozyten, die den Tumor infiltrieren, enthalten in der Entnahme von Hautknoten im Transit, einem Lymphknoten oder einer Metastase, dann die Stimulation der Lymphozyten, die den Tumor infiltrieren, resultierend aus dem Emergenzschritt, dann schließlich die Amplifikation der stimulierten Lymphozyten, die den Tumor infiltrieren, um mindestens eine Probe von Lymphozyten, die den Tumor infiltrieren, zu erhalten
ii) Mischung mindestens einer in Schritt i) erhaltenen Probe mit einer Zusammensetzung, umfassend in einem physiologisch verträglichen Medium:
a) humanes Serumalbumin in einer Menge zwischen 2 und 10 Gewichts-%,
b) mindestens ein Saccharid in einer Konzentration zwischen 0,05 M und 1 M und
c) DMSO und L-Cystein in einer Menge zwischen 2 und 15 Gewichts-% beziehungsweise in einer Konzentration zwischen 0,05 mM und 5 mM, oder
Coenzym Q10 und Propan-1,2-diol in einer Menge zwischen 0,005 und 1 Gewichts-% beziehungsweise in einer Menge zwischen 3 und 10 Volumen-%, dann
iii) Einfrieren der in Schritt ii) erhaltenen Mischung.

2. Verfahren zur Kryokonservierung nach Anspruch 1, **dadurch gekennzeichnet, dass** das Saccharid ausgewählt ist aus den Monosacchariden, den Disacchariden und den Trisacchariden.

3. Verfahren zur Kryokonservierung nach Anspruch 2, **dadurch gekennzeichnet, dass** das Saccharid ein Disaccharid ist, das aus Trehalose und Saccharose ausgewählt ist.

4. Verfahren zur Kryokonservierung nach einem der Ansprüche 1 bis 3, **dadurch gekennzeichnet, dass** das humane Serumalbumin in einer Menge zwischen 2,5 und 6 Gewichts-% vorliegt.

5. Verfahren zur Kryokonservierung nach einem der Ansprüche 1 bis 4, **dadurch gekennzeichnet, dass** das Saccharid in einer Konzentration zwischen 0,07 M und 0,5 M vorliegt.

6. Verfahren zur Kryokonservierung nach einem der Ansprüche 1 bis 5, **dadurch gekennzeichnet, dass** die in Schritt ii) verwendete Zusammensetzung umfasst:
a) humanes Serumalbumin, in einer Menge zwischen 2,5 und 6 Gewichts-%,
b) Trehalose, in einer Konzentration zwischen 0,07 M und 0,5 M, und
c) DMSO und L-Cystein, in einer Menge zwischen 2 und 15 Gewichts-% beziehungsweise in einer Konzentration zwischen 0,5 mM und 2 mM.

7. Verfahren zur Kryokonservierung nach einem der Ansprüche 1 bis 5, **dadurch gekennzeichnet, dass** die in Schritt ii) verwendete Zusammensetzung umfasst:
a) humanes Serumalbumin, in einer Menge zwischen 2,5 und 6 Gewichts-%,
b) Trehalose, in einer Konzentration zwischen 0,07 M und 0,5 M, und
c) Coenzym Q10 und Propan-1,2-diol, in einer Menge zwischen 0,007 und 0,1 Gewichts-% beziehungsweise in einer Menge zwischen 3 und 10 Volumen-%.

8. Verfahren zur Kryokonservierung nach einem der Ansprüche 1 bis 7, **dadurch gekennzeichnet, dass** das Einfrieren iii) bis zu einer Temperatur zwischen -100 °C und - 180 °C, vorzugsweise zwischen -140 °C und -160 °C durchgeführt wird.

9. Verfahren zur Kryokonservierung nach einem der Ansprüche 1 bis 8, **dadurch gekennzeichnet, dass** das Einfrieren iii) durchgeführt wird, indem die in ii) erhaltene Mischung in einen Behälter gegeben wird, der in eine Mischung aus Isopropylalkohol bei + 4 °C eingetaucht ist, wobei das Ganze auf eine Temperatur zwischen -70 °C und -100 °C gebracht wird.

10. Verfahren zur Kryokonservierung nach einem der Ansprüche 1 bis 9, **dadurch gekennzeichnet, dass** das Einfrieren iii) unter Verwendung einer programmierten Gefriervorrichtung durchgeführt wird.

11. Verwendung einer Zusammensetzung, umfassend in einem physiologisch verträglichen Medium:
a) humanes Serumalbumin in einer Menge zwischen 2 und 10 Gewichts-%,
b) mindestens ein Saccharid in einer Konzentration zwischen 0,05 M und 1 M und
c) DMSO und L-Cystein in einer Menge zwischen 2 und 15 Gewichts-% beziehungsweise in einer Konzentration zwischen 0,05 mM und 5 mM, oder
Coenzym Q10 und Propan-1,2-diol in einer Menge zwischen 0,005 und 1 Gewichts-% beziehungsweise in einer Menge zwischen 3 und 10 Volumen-%, zur Kryokonservierung von mindestens einer Probe von Lymphozyten, die den Tumor infiltrieren,
wobei die Probe durch *in vitro* Kultur von Lymphozyten, die den Tumor infiltrieren, aus einer Entnahme von Hautknoten im Transit, einem Lymphknoten oder einer Metastase eines Patienten, der an einem Melanom im Stadium 3 oder 4 leidet, erhalten ist, wobei die Kultur die Emergenz der Lymphozyten, die den Tumor infiltrieren, enthalten in der Entnahme von Hautknoten im Transit, einem Lymphknoten oder einer Metastase, umfasst, dann die Stimulation der Lymphozyten, die den Tumor infiltrieren, resultierend aus dem Emergenzschritt, dann schließlich die Amplifikation der stimulierten Lymphozyten, die den Tumor infiltrieren.

12. Verfahren zur Kryokonservierung nach einem der Ansprüche 1 bis 10 oder Verwendung nach Anspruch 11, bei dem/der die Stimulation der Lymphozyten, die den Tumor infiltrieren, resultierend aus dem Emergenzschritt, in Gegenwart von mindestens bestrahlten nicht proliferierenden allogenen Feederzellen durchgeführt wird.

13. Verfahren zur Kryokonservierung nach einem der Ansprüche 1 bis 10 oder 12 oder Verwendung nach Anspruch 11 oder 12, bei dem/der die Stimulation der Lymphozyten, die den Tumor infiltrieren, resultierend aus dem Emergenzschritt, durch Co-Kultur mit einer Mischung durchgeführt wird, gebildet aus mononukleären Zellen, die aus Blut eines gesunden humanen Spenders stammen, und B-Zellen, die mit dem Epstein-Barr-Virus, vorzugsweise der LAZ388-Linie, transformiert sind, alle nicht proliferieren.

## Claims

1. Method for the cryopreservation of at least one sample of tumour-infiltrating lymphocytes, comprising the following steps:
i) *in vitro* culture of tumour-infiltrating lymphocytes from a sample of in-transit cutaneous nodules, ganglion or metastasis coming from a patient presenting with a stage 3 or 4 melanoma, comprising the emergence of said tumour-infiltrating lymphocytes contained in the sample of in-transit cutaneous nodules, ganglion or metastasis, then the stimulation of the tumour-infiltrating lymphocytes resulting from the emergence step, and then finally the amplification of the tumour-infiltrating lymphocytes stimulated, in order to obtain at least one sample of tumour-infiltrating lymphocytes,
ii) the mixing of at least one sample obtained at step i) with a composition comprising, in a physiological acceptable medium:
a) human serum albumin in a quantity of between 2 and 10% by weight,
b) at least one saccharide in a concentration of between 0.05 M and 1 M, and
c) DMSO and L-cysteine, respectively in a quantity of between 2 and 15% by weight and in a concentration of between 0.05 mM and 5 mM, or
coenzyme Q10 and propane-1,2-diol, respectively in a quantity of between 0.005 and 1% by weight and in a quantity of between 3 and 10% by volume, and then
iii) freezing of the mixture obtained at step ii).

2. Cryopreservation method according to claim 1, **characterised in that** the saccharide is chosen from monosaccharides, disaccharides and trisaccharides.

3. Cryopreservation method according to claim 2, **characterised in that** the saccharide is a disaccharide chosen from trehalose and saccharose.

4. Cryopreservation method according to one of claims 1 to 3, **characterised in that** the human serum albumin is present in a quantity of between 2.5 and 6% by weight.

5. Cryopreservation method according to one of claims 1 to 4, **characterised in that** the saccharide is present in a concentration of between 0.07 M and 0.5 M.

6. Cryopreservation method according to one of claims 1 to 5, **characterised in that** the composition used at step ii) comprises:
a) human serum albumin, in a quantity of between 2.5 and 6% by weight,
b) trehalose, in a concentration of between 0.07 M and 0.5 M, and
c) DMSO and L-cysteine, respectively in a quantity of between 2 and 15% by weight and in a concentration of between 0.05 mM and 2 mM.

7. Cryopreservation method according to one of claims 1 to 5, **characterised in that** the composition used at step ii) comprises:
a) human serum albumin, in a quantity of between 2.5 and 6% by weight,
b) trehalose, in a concentration of between 0.07 M and 0.5 M, and
c) coenzyme Q10 and propane-1,2-diol, respectively in a quantity of between 0.007 and 0.1% by weight, and in a quantity of between 3 and 10% by volume.

8. Cryopreservation method according to one of claims 1 to 7, **characterised in that** the freezing iii) is performed to a temperature of between -100°C and -180°C, preferably between -140°C and -160°C.

9. Cryopreservation method according to one of claims 1 to 8, **characterised in that** the freezing iii) is performed by placing the mixture obtained at ii) in a receptacle immersed in a mixture of isopropyl alcohol at +4°C, the whole being put to a temperature of between -70°C and -100°C.

10. Cryopreservation method according to one of claims 1 to 9, **characterised in that** the freezing iii) is performed by means of a programmed freezer.

11. Use of a composition comprising, in a physiological acceptable medium:
a) human serum albumin, in a quantity of between 2 and 10% by weight,
b) at least one saccharide in a concentration of between 0.05 M and 1 M, and
c) DMSO and L-cysteine, respectively in a quantity of between 2 and 15% by weight and in a concentration of between 0.05 mM and 5 mM, or
coenzyme Q10 and propane-1,2-diol, respectively in a quantity of between 0.005 and 1% by weight, and in a quantity of between 3 and 10% by volume, for the cryopreservation of at least one sample of tumour-infiltrating lymphocytes,
said sample being obtained by *in vitro* culture of tumour-infiltrating lymphocytes using a sample of in-transit cutaneous nodules, ganglion or metastasis coming a patient presenting with a stage 3 or 4 melanoma, said culture comprising the emergence of said tumour-infiltrating lymphocytes contained in the sample of in-transit cutaneous nodules, ganglion or metastasis, then the stimulation of the tumour-infiltrating lymphocytes resulting from the emergence step, and then finally the amplification of the tumour-infiltrating lymphocytes stimulated.

12. Cryopreservation method according to one of claims 1 to 10, or the use according to claim 11, wherein the stimulation of the tumour-infiltrating lymphocytes resulting from the emergence step is done in the presence at least of non-proliferating irradiated allogenic feeder cells.

13. Cryopreservation method according to one of claims 1 to 10 or 12, or the use according to claim 11 or 12, wherein the stimulation of the tumour-infiltrating lymphocytes resulting from the emergence step is done by co-culture with a mixture formed from mononucleated cells coming from healthy human donor blood and B cells transformed by the Epstein Barr virus, preferably the LAZ388 line, all non-proliferating.
